# EUROPEAN PATENT SPECIFICATION

(11) **EP 1 913 396 B1**
(45) Date of publication and mention of the grant of the patent: **29.09.2010**
(21) Application number: 06760823.2
(22) Date of filing: 08.08.2006
(51) Int. Cl.: G01N 33/574, G01N 33/92

(54) **METHOD FOR DIAGNOSING TUMORS BY MEASURING THE LEVEL OF APOLIPOPROTEIN A-IV**
VERFAHREN ZUR DIAGNOSE VON TUMOREN DURCH MESSUNG DES NIVEAUS VON APOLIPOPROTEIN A-IV
METHODE PERMETTANT DE DIAGNOSTIQUER DES TUMEURS, CONSISTANT A MESURER LE TAUX D'APOLIPOPROTEINE A-IV

(30) Priority: 08.08.2005 AT 13332005
(43) Date of publication of application: 23.04.2008
(73) Proprietor: Vitateq Biotechnology GmbH, 6020 Innsbruck (AT)
(72) Inventor: DIEPLINGER, Hans, A-6020 Innsbruck (AT); KRONENBERG, Florian, A-6020 Innsbruck (AT)
(74) Representative: Sonn & Partner Patentanwälte
(86) International application number: PCT/AT2006/000336
(87) International publication number: WO 2007/016716

(56) References cited:
- WO-A-97/10503
- WO-A-2005/038461
- US-A1- 2005 059 013
- ROSSENEU M ET AL: "QUANTIFICATION OF HUMAN APOLIPOPROTEIN A-IV BY SANDWICH-TYPE ENZYME-LINKED IMMUNOSORBENT ASSAY" CLINICAL CHEMISTRY, AMERICAN ASSOCIATION FOR CLINICAL CHEMISTRY, WASHINGTON, DC, US, vol. 34, no. 4, 1988, pages 739-743, XP009008741 ISSN: 0009-9147
- KRONENBERG F ET AL: "LOW APOLIPOPROTEIN A-IV PLASMA CONCENTRATIONS IN MEN WITH CORONARY ARTERY DISEASE" JOURNAL OF THE AMERICAN COLLEGE OF CARDIOLOGY, ELSEVIER, NEW YORK, NY, US, vol. 36, no. 3, September 2000 (2000-09), pages 751-757, XP001146847 ISSN: 0735-1097
- KRONENBERG FLORIAN ET AL: "Apolipoprotein A-IV serum concentrations are elevated in patients with mild and moderate renal failure" JOURNAL OF THE AMERICAN SOCIETY OF NEPHROLOGY, vol. 13, no. 2, February 2002 (2002-02), pages 461-469, XP002406439 ISSN: 1046-6673 cited in the application
- NAVARRO M A ET AL: "Immune-regulation of the apolipoprotein A-I/C-III/A-IV gene cluster in experimental inflammation" CYTOKINE, ACADEMIC PRESS LTD, PHILADELPHIA, PA, US, vol. 31, no. 1, 7 July 2005 (2005-07-07), pages 52-63, XP004936872 ISSN: 1043-4666

## Description

The present invention relates to a method for diagnosing a tumor in an individual.

Human apolipoprotein A-IV (ApoA-IV; Swiss-Prot No. P06727) is a 46 kD plasma glycoprotein synthesized by the small intestine and to a much smaller extent by the liver. Mean plasma concentrations of ApoA-IV in humans are about 15 mg/dl (ranging from 10 to 18 mg/dl). Studies in rats showed that ApoA-IV is released by the small intestine into the mesenteric lymph and enters the plasma compartment as a structural protein of chylo-microns, very low-density lipoprotein (VLDL), high-density lipoprotein (HDL), or unassociated with lipoproteins. Although most glycoproteins have relatively long half-lives in plasma, human ApoA-IV is among the apolipoproteins with the highest turnover rates. Reports on the distribution of ApoA-IV in human plasma are contradictory. Depending on the used technique they describe ApoA-IV distributions ranging from ApoA-IV almost entirely bound to HDL to mostly unassociated with major lipoprotein fractions.

Various physiologic functions have been proposed for this apolipoprotein. Numerous in vitro studies suggested that it participates in several steps of the reverse cholesterol transport pathway, which removes and transports cholesterol from peripheral cells to the liver or steroidogenic organs for final conversion into bile acids or hormones. ApoA-IV enhances the formation of small HDL particles by activating the enzyme lecithin cholesterol acyltransferase (LCAT). It also modulates the activation of lipoprotein lipase and the cholesteryl ester transfer protein (CETP)-mediated transfer of cholesteryl esters from HDL to LDL in in vitro studies. In line with these in vitro data, overex-pression of ApoA-IV in transgenic mice led to reduced aortic lesions and protection against atherosclerosis. These results were recently confirmed by case-control studies in three independent human populations: ApoA-IV plasma concentrations were found to be significantly lower in patients with coronary artery disease as compared to control groups (KRONENBERG F, et al. J Am Coll Cardiol 36:751-757, 2000); a correlation between plasma ApoA-IV concentrations and atherosclerosis was also observed in patients with kidney failure (KRONENBERG F, et al. J Am Soc Nephrol 13:461-469, 2002).

The US 2002/068319 A discloses the identification of several human secretory proteins, which show a high homology to ApoA-IV.

In the US 2005/0059013 A1 a method for diagnosing ovarian cancer by determining the amount of ApoA-I in a sample is described.

The JP 2004333274 A relates to a method for determining apolipoprotein A2 in a serum sample of individuals suffering from cancer.

The WO 97/10503 discloses a method for diagnosing prostate cancer by determining the amount of apoliprotein D.

The EP 1 560 024 A1 relates to a method for diagnosing intestinal diseases by determining the amount of ApoA-IV in the serum or plasma of an individual. '

In the DE 103 43 815 A1 the use of apoliprotein D1 as tumor marker is disclosed.

Several studies suggest a role of the kidney in metabolism of ApoA-IV. Patients with chronic kidney disease exhibit markedly elevated ApoA-IV concentrations (KRONENBERG F, et al. Kidney Int (Suppl):5113-5116, 2003). ApoA-IV starts to increase already in the earliest phases of kidney insufficiency, which identifies ApoA-IV as an early marker of kidney impairment (KRONENBERG F, et al. J Am Soc Nephrol 13:461-469, 2002). Although the liver was found to be the major site of degradation, the kidney significantly contributes to catabolism of ApoA-IV.

Although the ApoA-IV levels in individuals suffering from kidney diseases were already intensively studied there are no data available how said levels are influenced by the presence of other diseases like tumors, in particular solid tumors, in an individual. Since it is of major importance in the medical diagnosis to have tumor markers which may allow the detection of a tumor in an individual, especially at an early stage of development, in a fast and accurate manner, it is an object of the present invention to provide a method and a means for diagnosing a tumor in an individual and for monitoring the efficiency of the treatment of said tumor (e.g. after surgical removal of a solid tumor), especially monitoring the recurrence of a tumor.

Therefore, the present invention provides a method for diagnosing a tumor in an individual comprising the following steps:
- measuring the amount of apolipoprotein A-IV (ApoA-IV) in a body fluid or tissue sample of an individual,
- comparing the.measured amount of ApoA-IV in said sample with a reference value, wherein the reference value is the ApoA-IV content in a sample derived from an.individual without a tumor, and
- diagnosing a tumor if the ApoA-IV content in the sample is decreased compared to the reference value.

It could surprisingly be shown that the determination of the amount of ApoA-IV in a sample of an individual allows diagnosing the presence of a solid tumor in said individual. If, for instance, the Apo-IV level in the serum of an individual is lower than the average amount of ApoA-IV in individuals lacking a tumor, the presence of a tumor can be diagnosed.

"Reference value" refers to the average amount of ApoA-IV in healthy individuals which are not suffering from a tumor. The reference value is preferably calculated from at least one, preferably at least five, more preferably at least ten, even more preferably at least 50 samples of, preferably, different individuals. It is noted that the ApoA-IV serum level depends also on the presence of other diseases like renal impairment (increased ApoA-IV level compared to healthy individuals not suffering from renal impairment), coronary artery diseases and atherosclerotic complications (decreased ApoA-IV level compared to healthy individuals not suffering from coronary artery diseases and atherosclerotic complications) (see EP 1 410 039 A). Therefore, the reference value is preferably calculated from individuals not suffering from a turmor, renal impairment, coronary artery diseases and atherosclerotic complications

According to a preferred embodiment of the present invention the individual is human.

Of course the method according to the present invention may also be applied in mammals, especially in pets (e.g. dogs, cats) and farm animals (e.g. horses, cattle, sheep). In case where the ApoA-IV level is measured in other mammals than humans the ApoA-IV level in healthy mammals has to be determined before the method according to the present invention is used to diagnose a tumor.

The tumor to be diagnosed is preferably selected from the group consisting of renal tumor and tumors of the reproductive organs (i.e. the reproductive system), especially ovarian carcinoma, cervical cancer and testicular carcinoma.

It turned out that especially in patients suffering from renal tumor and tumors of the reproductive organs (e.g. ovarian carcinoma, cervical cancer and testicular carcinoma) (or of more than one of said tumors) the ApoA-IV serum level was decreased compared to the serum levels of individuals not suffering from said diseases and could already be proven as reliable diagnostic marker in the clinical practice. The present invention is, however, also applicable to tumors, especially solid tumors, e.g. tumors involving epithelial cells.

Tumours in the reproductive organs, in particular ovarian and testicular tumours, specifically affect young people (also a large group of people between 30 and 50). In most of these tumours, early diagnosis is paramount for survival. The reproductive system includes the gonads - male testes and female ovaries and other accessory ducts and glands (gonos = seed). These provide the means for reproduction, the continuation of the species, and passing on of genetic material to the next generation.

The method according to the present invention has also proven to be effective for the diagnosis of cervical cancer, especially already in early stages of this type of cancer (e.g. pre-stage 0, stage 0, I, IA or IB).

Staging of cervical cancer has been developed to describe the extent of cancer growth. The stage of cervical cancer describes the tumor's size, depth of penetration within the cervix and spread within and beyond the cervix. Cervical cancer staging is usually described in terms of the FIGO system, a staging scheme developed by the International Federation of Gynecology and Obstetrics. The FIGO classifications are grouped within basic stages labeled stage 0 through stage IV (0-4):
* Stage 0 or carcinoma in situ is very early cancer. The abnormal cells are found only in the first layer of cells of the lining of the cervix and do not invade the deeper tissues of the cervix.
* Stage I cancer involves the cervix but has not spread nearby.
* Stage IA indicates a very small amount of cancer that is only visible under a microscope is found deeper in the tissues of the cervix.
* Stage IB indicates a larger amount of cancer is found in the tissues of the cervix.
* Stage II cancer has spread to nearby areas but is still inside the pelvic area.
* Stage IIA cancer has spread beyond the cervix to the upper two-thirds of the vagina.
* Stage IIB cancer has spread to the tissue around the cervix.
* Stage III cancer has spread throughout the pelvic area. Cancer cells may have spread to the lower part of the vagina. The cells also may have spread to block the tubes that connect the kidneys to the bladder.
* Stage IV cancer has spread to other parts of the body.
* Stage IVA cancer has spread to the bladder or rectum (organs close to the cervix).
* Stage IVB cancer has spread to other organs such as the lungs.

Despite the ability to reduce the incidence of cervical cancer significantly by early detection (especially prior to stage o (pre-stage 0)), there are still limitations currently in the screening process for this disease. Most dominant is the fact that many patients are not aware of the currently available screening methods. Detecting cervical cancer in serum samples within routineously performed serum analysis would solve this problem. Present screening procedures are not able to detect CIN and/or cervical cancer in serum samples, they are designed for the analysis of cervical cells. All the screening procedures established thus far have their specific limitations and none of these methods allows the detection of CIN or cervical cancer in samples other than derived from the transforming region of the cervix. Furthermore, each of these screening programs is far beyond the possibility to be used for a population screening especially in developing countries. This situation would change dramatically with the development of precise, rapid, and inexpensive CIN and/or cervical cancer diagnostic tools.

Of course the method according to the present invention enables the person skilled in the art to diagnose the presence not only of one tumor but of a multiplicity of tumors in an indi-vidual. Therefore, it is also possible to diagnose a tumor in an individual suffering from more than one tumor, although here the final diagnosis should carefully consider these circumstances and can, as for all final diagnostic decisions, only be made by the responsible doctor.

Although the amount of ApoA-IV may be determined in several body fluids and tissue samples, it is preferred that the body fluid is blood, serum or fractions thereof.

It is advantageous that a method for diagnosing a tumor employs samples which can be obtained in a relatively simple and fast way without being as invasive as, for instance, a surgical intervention (e.g. biopsy). Therefore, a tumor marker may be preferably detected in blood, serum or fractions thereof. Also when the method according to the present invention will be used to monitor the progress of a tumor treatment, the sample has to be obtained by a method which is preferably not invasive because it is not possible to obtain, for instance, tissue samples in a sufficient amount required to monitor the progress of the treatment.

According to a preferred embodiment of the present invention the reference value is between 10 and 18 mg, preferably 13 to 15 mg, ApoA-IV/dL serum.

The comparison of the measured value in the individual to the diagnosed with the present invention and the reference value is also dependent from the body fluid or tissue wherefrom the values are taken and measured. Preferably, of course, serum is tested, wherein the (healthy) reference value is usually between 10 and 18 mg ApoA-IV/dL serum, if tested with ApoA-IV ELISA. However, as stated above, these absolute values are always dependent on the very specific way of measuring the amount of ApoA-IV and the body fluid/tissue wherein ApoA-IV amounts are measured. Other preferred sources than blood derived body fluids of material to be tested according to the present invention include tumor tissue, especially biopsy material from tumor tissue.

The tumor is diagnosed when the level of ApoA-IV is lower than 10 mg, preferably lower than 8 mg, ApoA-IV/dL serum.

According to a preferred embodiment of the present invention the ApoA-IV content in the sample is considered to be decreased, if it is at least 10 % lower, preferably at least 30 % lower, especially at least 50 % lower, than the ApoA-IV content in a sample from an individual without a tumor.

A decreased ApoA-IV level is regarded as a level being lower than in a healthy control individual not suffering from a tumor.

The amount of ApoA-IV in the sample is preferably determined with an immunochemical method employing antibodies directed to ApoA-IV.

The determination of an amount of a protein in a sample can be carried out, for instance, by immunochemical methods. Said methods employ antibodies which are directed to the protein or fragments thereof to be measured.

The antibodies are preferably monoclonal antibodies.

The antibodies which can be used in the method according to the present invention may be polyclonal or monoclonal. However, it is preferred to employ in said method monoclonal antibodies. Monoclonal antibodies directed to an antigen of ApoA-IV may be obtained by methods known in the art (e.g. Köhler G and Milstein C. Nature (1975) 256:495-457). Also methods for the production of polyclonal antibodies are well known to a person skilled in the art. Such antibodies may be obtained by administering to a mammal, preferably to a rabbit, mouse or sheep, an antigen, optionally in combination with at least one adjuvant, and to isolate from the serum obtained the fraction comprising the antibodies.

The antibody directed to ApoA-IV or fragment thereof comprises preferably a detection marker, preferably a chromogenic, fluorogenic or radioactive marker.

In order to detect an antibody in an assay it is necessary to label, e.g., the antibody directed to ApoA-IV with an appropriate marker which can easily be detected by detection means (e.g. photomultiplier). Of course it is also possible that the antibody directed to ApoA-IV is not directly labelled. In an assay involving such antibodies those antibodies which are directed to the ApoA-specific antibodies have to be labelled. Which of those antibodies have to be labelled depends mainly on the assay used to determine ApoA-IV in the serum.

According to a preferred embodiment of the present invention the immunochemical method is selected from the group consisting of Western blot, enzyme-linked immunosorbent assay (ELISA) and radio immuno assay (RIA). The Western blot is used for monitoring the specificity of the used monoclonal and/or polyclonal antibodies for the antigen apolipoprotein A-IV.

Among the immunochemical methods known in the art Western blot, enzyme-linked immunosorbent assay (ELISA) or radio immuno assay (RIA) are preferably used. ELISA and RIA may be used as competitive assays and ELISA as sandwich assay (e.g. Rosseneu M, et al. Clin Chem (1988) 34:739-743), too.

Another aspect of the present invention relates to the use of a method according to the present invention for monitoring the efficiency of a treatment of a tumor.

The reduced amount of ApoA-IV in an individual suffering from a tumor, in particular in the serum of said individual, allows the monitoring of the ApoA-IV levels in the course of a treatment of a tumor. Since the ApoA-IV serum level depends significantly from the presence of a tumor in an individual, the increase of ApoA-IV serum levels to a reference value indicates a successful treatment of a cancer patient. In order to monitor the change in ApoA-IV serum levels during treatment, samples of the individual are taken prior the treatment (e.g. prior removal of the tumor) and in defined intervals in the course of the treatment (e.g. chemotherapy). Of course, this method may also be used in a long term examination to monitor a tumor relapse in an individual.

The tumor is preferably selected from the group consisting of renal tumor and tumors of the reproductive organs, especially ovarian carcinoma, cervical cancer and testicular carcinoma.

Another aspect of the present invention relates to a kit for diagnosing a tumor in a patient or for monitoring the efficiency of a treatment of a tumor comprising one or more of the following components:
- a positive control from an individual suffering from a tumor,
- means for measuring the amount of apolipoprotein A-IV (ApoA-IV) in a body fluid sample or tissue sample of an individual, and
- a sample from an individual not suffering from said tumor as reference value means.

The positive control from an individual suffering from a tumor and the sample from an individual not suffering from said tumor are preferably stabilised, preferably stabilised by lyophilisation.

Samples comprising biological material tend to be instable. Therefore, if such samples and positive controls are provided in a kit, said samples and positive controls have to be stabilised in order to allow the storage of said kit for a prolonged time at temperatures less than 6°C or at room temperature. If the kit is intended to be stored at room temperature it is necessary that the biological material contained in said kit is e.g. lyophilised. Lyophilisation is a wide spread method to conserve proteinous samples. If the kit is intended to be stored under 6°C, preferably under 0°C, the biological material has to be provided in a solution which prevents the deterioration of the proteins contained in said biological material. Said solution may comprise for instance glycerol, mercapto-ethanol and preservatives.

The means for measuring the amount of ApoA-IV are selected from anti-ApoA-IV antibodies, especially polyclonal antibodies, secondary antibodies, especially enzymatically or chemically labelled secondary antibodies, ApoA-IV-RNA specific nucleic acids, ApoA-IV specific enzymatic tests, ApoA-IV specific ELISAs, or combinations thereof.

The amount of ApoA-IV in sample can be determined by several methods, whereby methods employing antibodies as means for measuring the amount are preferably used.

The present invention is further illustrated by the following figures and examples, without being restricted thereto.

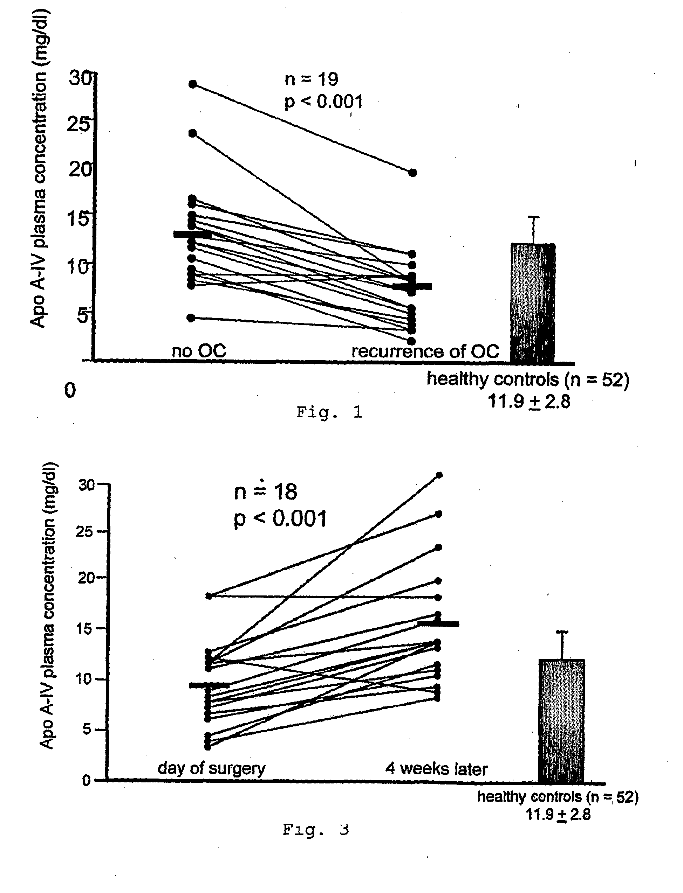

Fig. 1 shows the change of the ApoA-IV plasma levels of tumor patients whose tumor was removed by surgery. After said surgical intervention the ApoA-IV levels show such levels which are normally measured in healthy individuals (no ovarian carcinoma; no OC). In those patients were the ovarian carcinoma recurred the ApoA-IV levels decreased significantly (recurrence of OC) under those ApoA-IV levels of healthy individuals ("reference value"). This shows that with the method according to the present invention the recurrence of a tumor can be monitored.

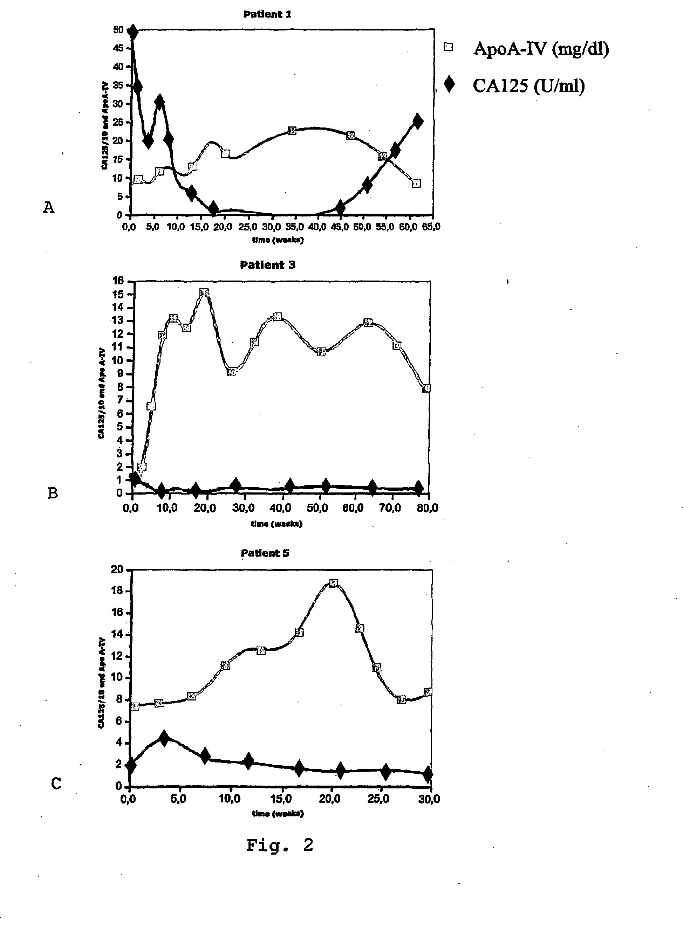

Fig. 2A to 2F show comparative results between the ApoA-IV plasma levels and the levels of CA125, a well known tumor marker, found in blood of ovarian carcinoma patients.
Fig. 3 shows ApoA-IV in patients suffering from testicular cancer.

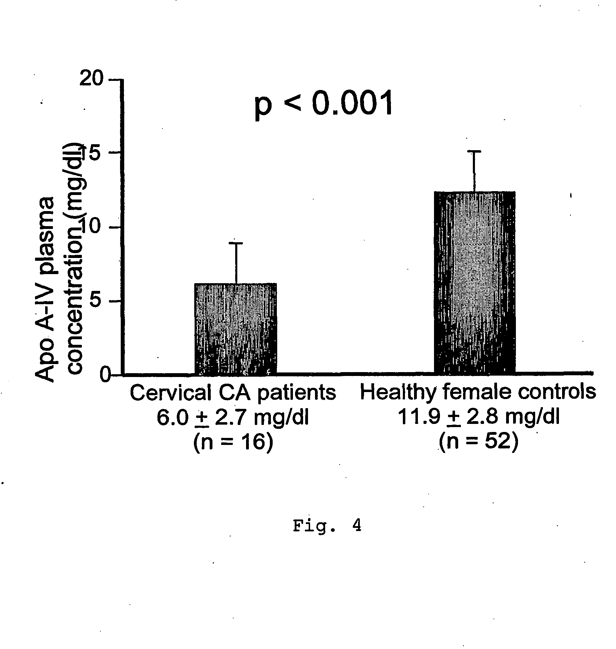

Fig. 4 shows plasma concentrations of Apo A-IV (mg/dl, means +/- standard deviation) of a population-based control group (n=52) and patients with cervical cancer (n=16) at various stages of FIGO at the day of surgical tumor removal (preoperative)

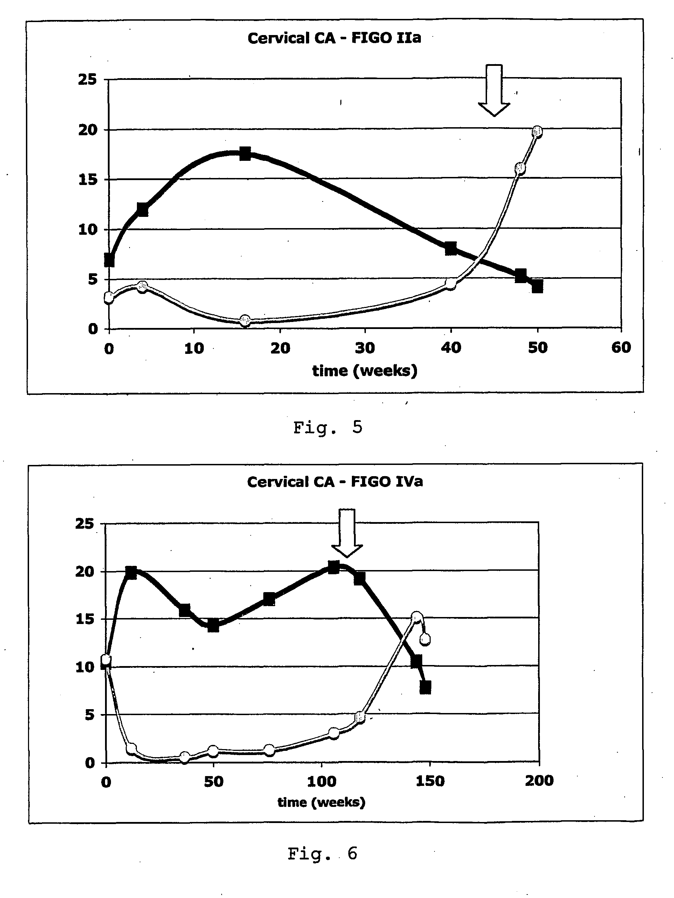

Fig. 5 shows plasma concentrations of Apo A-IV (squares) and the conventional tumor marker SCC (circles) of a representative patient with cervical cancer at FIGO stage IIa at the day of surgical tumor removal (day 0, preoperative) and after 50 weeks of observation until tumor recurrence (arrow). Reduced plasma concentrations of Apo A-IV increase to normal levels and decrease again in the case of tumor recurrence.
Fig. 6 shows plasma concentrations of Apo A-IV (squares) and the conventional tumor marker SCC (circles) of a representative patient with cervical cancer at FIGO stage IVa at the day of surgical tumor removal (day 0, preoperative) and after 150 weeks of observation until tumor recurrence (arrow). Reduced plasma concentrations of Apo A-IV increase to normal levels and decrease again in the case of tumor recurrence.

### EXAMPLES:

### EXAMPLE 1: ApoA-IV as marker for renal tumor

### Methods:

### Subjects

Human blood samples from 30 patients (18 male and 12 female) were included in this example. All patients were undergoing total unilateral nephrectomy due to kidney cell carcinoma. None of the patients had severe proteinuria (the dipstick test for proteinuria was 1+ positive at most).

A blood sample was taken after overnight fasting prior to surgery. Ethylenediaminetetraacetic acid (EDTA) plasma was obtained after low-speed centrifugation at 4°C and frozen at -80°C prior to use. It was ensured that patients did not undergo any examination with contrast agents up to 1 week before blood donation, which could influence kidney function.

### Measurement of plasma ApoA-IV concentration

Plasma ApoA-IV concentrations were determined using a double-antibody enzyme-linked immunosorbent assay (ELISA), which employs an affinity-purified polyclonal antihuman ApoA-IV antibody for coating and the same antibody coupled to horseradish peroxidase for detection. Plasma with known content of ApoA-IV served as calibration standard. The lower detection limit of this assay is 0.002 mg/dl (KRONENBERG F, et al. J Lipid Res 35:1318-1328, 1994). Each sample was analyzed in duplicate. The affinity-purified antibody was prepared by immunoadsorption chromatography of rabbit antihuman ApoA-IV antiserum using purified plasma ApoA-IV coupled to CNBr-activated sepharose Q2 4B (Amersham Pharmacia Biotech AB, Sweden). Anti-Apo A-IV serum was obtained by immunizing rabbits with purified plasma ApoA-IV (DIEPLINGER H, et al. Eur J Clin Invest 22:166-174, 1992).

### Immunoblot analysis

Immunoblot analysis of ApoA-IV was performed on samples separated by sodium dodecyl sulfatepolyacrylamide gel electro-phoresis (SDS PAGE) and transferred to cellulose nitrate membranes. Purified ApoA-IV was used as standard. ApoA-IV was isolated from human plasma by lipoprotein depletion, followed by incubation with a triglyceride-phospholipid emulsion and separation of hydrophobic, lipid-bound proteins by anion-exchange chromatography (STEINMETZ A, et al. J Chromatogr 487:154-160, 1989). The same affinity-purified, horseradish peroxidase-labeled antihuman ApoA-IV that was included in the ELISA protocol was also used for immunoblotting.

### Statistical evaluation

Mean concentrations of Apo A-IV between study groups were compared using the Mann-Whitney U test. Paired samples were compared using the Wilcoxon test

### Results:

### Specificity of ApoA-IV antibody

In order to verify the specificity of polyclonal rabbit antihuman ApoA-IV antibody used for immunohistochemistry the antibody was analysed by immunoblotting analysis of human kidney protein isolated from tissues from two different patients and corresponding plasma samples. ApoA-IV antibody was able to detect only one protein band in tissue and plasma samples, but a double band in the ApoA-IV preparation, which served as standard. Peptide sequencing confirmed that both protein bands of the standard represent ApoA-IV. The second, smaller ApoA-IV isoform might therefore result from deglycosylation or proteolytic degradation.

### ApoA-IV serum levels

Analysis of plasma samples revealed that the concentrations of ApoA-IV in plasma were generally lower in patients suffering from a renal tumor (6.8 ± 4.4 mg/dl)compared to healthy individuals (11.9 + 2.8 mg/dl, n=52) (EZEH B, et al. J Lipid Res 44:1523-1529, 2003, KRONENBERG F, et al. J Am Coll Cardiol 36:751-757, 2000).

### EXAMPLE 2: ApoA-IV as marker for ovarian carcinoma

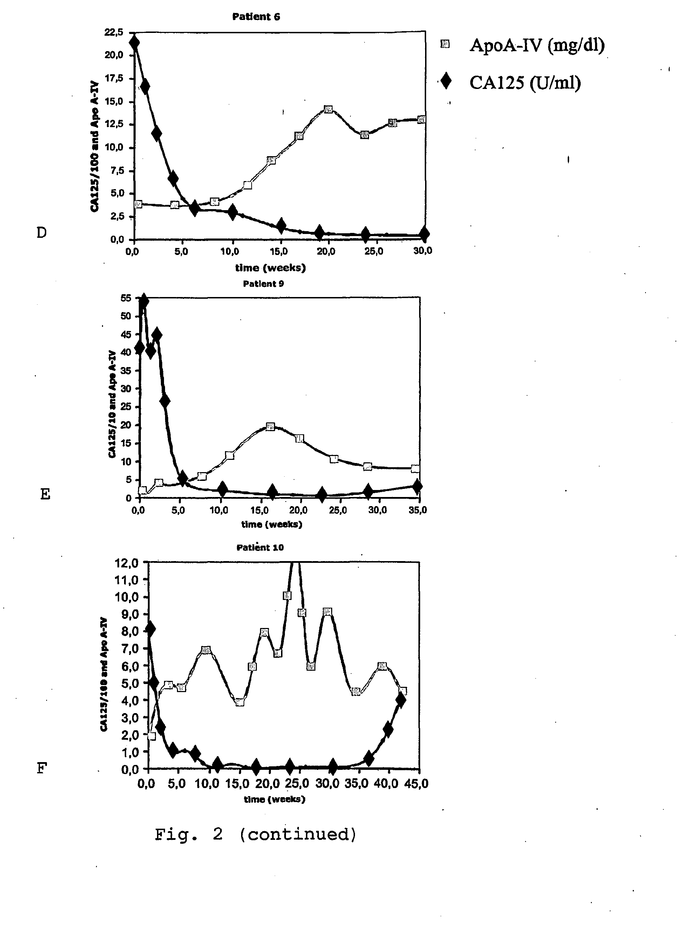

a) The first group of patients comprised 19 patients suffering from ovarian carcinoma. The recurrence of ovarian carcinoma was determined by the quantification of the ApoA-IV plasma levels in said patients. The initial ApoA-IV plasma level was determined approximately 30 days after the surgical intervention, showing that the patients' ApoA-IV plasma levels were near the reference value of a healthy individual. After recurrence of ovarian carcinoma the ApoA-IV plasma levels were decreased significantly compared to the initial values (Fig. 1).
b) In a second clinical trial the ApoA-IV plasma levels of 10 cancer patients before and after surgical intervention were determined over a period of time and compared to the plasma levels of CA125, a well known tumor marker. The CA125 as well as the ApoA-IV plasma levels were measured in ovarian carcinoma patients prior (time point 0) and after surgery for a period of at least 30 weeks. In Fig. 2A a correlation between both tumor markers is shown. High amounts of CA125, showing the presence of ovarian carcinoma, correlate with low ApoA-IV serum levels. A recurrence of ovarian carcinoma can be observed after 50 to 55 weeks after surgery. These results were confirmed by histological examinations. However, although the CA125 levels did not reveal the recurrence of ovarian carcinoma, ApoA-IV serum levels clearly show in two other patients the presence of ovarian carcinoma after 65 to 70 weeks after surgery (see Fig. 2B,C). These results were also confirmed by histological examinations. Therefore it could be clearly demonstrated that the ApoA-IV plasma levels are definitely better tumor, in particular ovarian carcinoma, markers than CA125, whose non-detection in this case resulted in a false negative diagnosis. In Fig. 2D the result of the CA125 and ApoA-IV determination of a successful treatment of a patient suffering from ovarian carcinoma could be shown. In Fig. 2E and 2F the correlation of CA125 and ApoA-IV levels in patients suffering from ovarian carcinoma could be confirmed.

### EXAMPLE 3: ApoA-IV as marker for testicular cancer

A group of 18 patients suffering from testicular cancer was examined. Results of ApoA-IV plasma levels are depicted in the following table.

**Table:**

| Plasma Apo A-IV (mg/dl) as tumor marker for testicular cancer | | | |
|---|---|---|---|
| Patient | Diagnosis | Time of surgery | 4 weeks later |
| 1 | S | 18,42 | 27,07 |
| 2 | S | 11,90 | 9,07 |
| 3 | S | 6,19 | 10,50 |
| 4 | NS | 7,69 | 14,42 |
| 5 | S | 12,70 | 23,98 |
| 6 | MT | 3,59 | 8,56 |
| 7 | MT | 8,51 | 15,38 |
| 8 | S | 6,34 | 8,42 |
| 9 | S | 11,70 | 31,34 |
| 10 | NS | 3,37 | 14,38 |
| 11 | NS | 7,68 | 13,26 |
| 12 | MT | 18,34 | 17,55 |
| 13 | S | 11,73 | 13,68 |
| 14 | S | 12,30 | 19,60 |
| 15 | S | 11,70 | 15,41 |
| 16 | MT | 4,93 | 10,76 |
| 17 | NS | 8,20 | 14,12 |
| 18 | NS | 7,60 | 10,60 |
| | | | |
| Mean | | 9,61 | 15,45 |
| SD | | 4,36 | 6,42 |

| | | | |
|---|---|---|---|
| S= Seminom NS = Non-Semlnom MT = Mixed Tumor | | | |

### EXAMPLE 4: ApoA-IV as marker for cervical cancer

Finally, also a group of patients with another tumor of reproductive organs, cervical cancer which is the 2nd most frequent cancer in females was investigated. No specific serum tumor marker exists at all for the identification of this frequent cancer. The conventionally used plasma marker SCC (Squamous cell carcinoma antigen) is again not specific enough for cervical cancer.

By the present invention not only significantly reduced plasma concentrations of ApoA-IV in 16 patients with cervical cancer could be shown (Fig. 4), but again the suitability of ApoA-IV as monitoring tumor marker in the longitudial design (n=16) could be demonstrated. ApoA-IV was decreased in all investigated FIGO stages of the cancer, increased to normal values of healthy controls after tumor removal and decreased again at tumor recurrence (Fig. 5 and Fig. 6).

### References:

KRONENBERG F, et al. J AmColl Cardiol 36:751-757, 2000
KRONENBERG F, et al. J Am Soc Nephrol 13:461-469, 2002
KRONENBERG F, et al. Kidney Int (Suppl):S113-S116, 2003
DIEPLINGER H, et al. Eur J Clin Invest 22:166-174, 1992
KRONENBERG F, et al. J AmSoc Nephrol 6:110-120, 1995
KRONENBERG F, et al. J Lipid Res 35:1318-1328, 1994
STEINMETZ A, et al. J Chromatogr 487:154-160, 1989
EZEH B, et al. J Lipid Res 44:1523-1529, 2003,

## Claims

1. Method for diagnosing a tumor in an individual comprising the following steps:
- measuring the amount of apolipoprotein A-IV (ApoA-IV) in a body fluid sample or tissue sample of an individual,
- comparing the measured amount of ApoA-IV in said sample with a reference value, wherein the reference value is the ApoA-IV content in a sample derived from an individual without a tumor, and
- diagnosing a tumor if the ApoA-IV content in the sample is decreased compared to the reference value.

2. Method according to claim 1, **characterised in that** said individual is human.

3. Method according to claim 1 or 2, **characterised in that** the tumor is selected from the group consisting of renal tumor and tumors of the reproductive organs, especially ovarian carcinoma, cervical cancer and testicular carcinoma.

4. Method according to any one of claims 1 to 3, **characterised in that** the body fluid sample is a blood sample, a plasma sample or a sample of a plasma fraction.

5. Method according to any one of claims 1 to 4, **characterised in that** said reference value is between 10 and 18 mg, preferably 13 to 15 mg, ApoA-IV/dL serum.

6. Method according to any one of claims 1 to 5, **characterised in that** the tumor is diagnosed when the level of ApoA-IV is lower than 10, preferably lower than 8, mg ApoA-IV/dL serum.

7. Method according to any one of claims 1 to 6, **characterised in that** the ApoA-IV content in the sample is decreased, if it is at least 10 % lower, preferably at least 30 % lower, especially at least 50 % lower, than the ApoA-IV content in a sample from an individual without a tumor.

8. Method according to any one of claims 1 to 7, **characterised in that** the amount of ApoA-IV in the sample is determined with an immunochemical method employing antibodies directed to ApoA-IV.

9. Method according to claim 8, **characterised in that** said antibodies are monoclonal antibodies.

10. Method according to claim 8 or 9, **characterised in that** said antibody comprises a detection marker, preferably a chromogenic, fluorogenic or radioactive marker.

11. Method according to any one of claims 8 to 10, **characterised in that** said immunochemical method is selected from the group consisting of Western Blot, enzyme-linked immunosorbent assay (ELISA) and radio immuno assay (RIA).

12. Use of a method according to any one of claims 1 to 11 for monitoring the efficiency of a treatment of a tumor.

13. Use according to claim 12, wherein said tumor is selected from the group consisting of renal tumor and tumor's of the reproductive organs, especially ovarian carcinoma, cervical cancer and testicular carcinoma.

14. Kit for diagnosing a tumor in a patient or for monitoring the efficiency of a treatment of a tumor comprising one or more of the following components:
- a positive control which is a sample from an individual suffering from a tumor,
- means for measuring the amount of ApoA-IV in a body fluid or tissue sample, said means for measuring the amount of ApoA-IV are selected from anti-ApoA-IV antibodies, especially polyclonal antibodies, secondary antibodies, especially enzymatically or chemically labelled secondary antibodies, ApoA-IV-RNA specific nucleic acids, ApoA-IV specific enzymatic tests, ApoA-IV specific ELISAs, or combinations thereof, and
- a sample from an individual not suffering from said tumor as reference value means.

15. Kit according to claim 14, **characterised in that** the positive control and the sample from an individual not suffering from said tumor are stabilised, preferably stabilised by lyophilisation.

## Patentansprüche

1. Verfahren zur Diagnostizierung eines Tumors in einem Individuum, umfassend die folgenden Schritte:
- Messen der Menge von Apolipoprotein A-IV (ApoA-IV) in einer Körperflüssigkeits- oder Gewebeprobe eines Individuums,
- Vergleichen der gemessenen Menge an ApoA-IV in dieser Probe mit einem Referenzwert, wobei der Referenzwert der ApoA-IV-Gehalt in einer Probe, die von einem Individuum ohne Tumor stammt, ist, und
- Diagnostizieren eines Tumors, wenn der ApoA-IV-Gehalt in der Probe im Vergleich zum Referenzwert verringert ist.

2. Verfahren nach Anspruch 1, **dadurch gekennzeichnet, dass** das Individuum ein Mensch ist.

3. Verfahren nach Anspruch 1 oder 2, **dadurch gekennzeichnet, dass** der Tumor ausgewählt ist aus der Gruppe bestehend aus Nierentumor und Tumoren der Fortpflanzungsorgane, insbesondere Ovarialkarzinom, Gebärmutterhalskrebs und Hodenkarzinom.

4. Verfahren nach einem der Ansprüche 1 bis 3, **dadurch gekennzeichnet, dass** die Körperflüssigkeitsprobe eine Blutprobe, eine Plasmaprobe oder eine Probe einer Plasma-Fraktion ist.

5. Verfahren nach einem der Ansprüche 1 bis 4, **dadurch gekennzeichnet, dass** der Referenzwert zwischen 10 und 18 mg, vorzugsweise 13 bis 15 mg ApoA-IV/dl Serum ist.

6. Verfahren nach einem der Ansprüche 1 bis 5, **dadurch gekennzeichnet, dass** der Tumor diagnostiziert wird, wenn der Grad an ApoA-IV niedriger als 10, vorzugsweise niedriger als 8 mg ApoA-IV/dl Serum ist.

7. Verfahren nach einem der Ansprüche 1 bis 6, **dadurch gekennzeichnet, dass** der ApoA-IV-Gehalt in der Probe verringert ist, wenn er um mindestens 10% niedriger, vorzugsweise mindestens 30% niedriger, insbesondere mindestens 50% niedriger als der ApoA-I-V-Gehalt in einer Probe von einem Individuum ohne Tumor ist.

8. Verfahren nach einem der Ansprüche 1 bis 7, **dadurch gekennzeichnet, dass** die Menge an ApoA-IV in der Probe mit einem immunochemischen Verfahren unter Verwendung von auf ApoA-IV gerichteten Antikörpern bestimmt wird.

9. Verfahren nach Anspruch 8, **dadurch gekennzeichnet, dass** die Antikörper monoklonale Antikörper sind.

10. Verfahren nach Anspruch 8 oder 9, **dadurch gekennzeichnet, dass** der Antikörper einen Detektionsmarker, vorzugsweise einen chromogenen, fluorogenen oder radioaktiven Marker, umfasst.

11. Verfahren nach einem der Ansprüche 8 bis 10, **dadurch gekennzeichnet, dass** das immunochemische Verfahren ausgewählt ist aus der Gruppe bestehend aus Western Blot, Enzyme-Linked Immunosorbent Assay (ELISA) und Radio-Immuno-Assay (RIA).

12. Verwendung eines Verfahrens nach einem der Ansprüche 1 bis 11 zur Überwachung der Effizienz einer Tumorbehandlung.

13. Verwendung nach Anspruch 12, wobei der Tumor ausgewählt ist aus der Gruppe bestehend aus Nierentumor und Tumoren der Fortpflanzungsorgane, insbesondere Ovarialkarzinom, Gebärmutterhalskrebs und Hodenkarzinom.

14. Set zur Diagnostizierung eines Tumors bei einem Patienten oder zur Überwachung der Effizienz einer Tumorbehandlung, umfassend einen oder mehrere der folgenden Komponenten:
- eine positive Kontrolle, welche eine Probe von einem Individuum ist, das an einem Tumor leidet,
- Mittel zum Messen der Menge des ApoA-IV in einer Körperflüssigkeits- oder Gewebeprobe, wobei besagte Mittel zum Messen der Menge an ApoA-IV ausgewählt sind aus Anti-ApoA-IV-Antikörpern, insbesondere polyklonalen Antikörpern, sekundären Antikörpern, insbesondere enzymatisch oder chemisch markierten sekundären Antikörpern, ApoA-IV-RNA-spezifischen Nukleinsäuren, ApoA-IV-spezifischen enzymatischen Tests, ApoA-IV-spezifischen ELISAs oder Kombinationen daraus, und
- eine Probe von einem Individuum, das nicht an diesem Tumor leidet, als Referenzwert-Mittel.

15. Set nach Anspruch 14, **dadurch gekennzeichnet, dass** die positive Kontrolle und die Probe von einem Individuum, das nicht an diesem Tumor leidet, stabilisiert sind, vorzugsweise durch Lyophilisation stabilisiert sind.

## Revendications

1. Méthode pour le diagnostic d'une tumeur chez un individu, comprenant les étapes suivantes :
- mesure de la quantité d'apolipoprotéine A-IV (ApoA-5IV) dans un échantillon de fluide corporel ou échantillon de tissu d'un individu,
- comparaison de la quantité mesurée d'ApoA-IV dans ledit échantillon avec une valeur de référence, la valeur de référence étant la teneur en ApoA-IV d'un échantillon provenant d'un individu dépourvu de tumeur, et
- le diagnostic d'une tumeur si la teneur en ApoA-IV de l'échantillon est réduite, comparativement à la valeur de référence.

2. Méthode suivant la revendication 1, **caractérisée en ce que** ledit individu est un être humain.

3. Méthode suivant la revendication 1 ou 2, **caractérisée en ce que** la tumeur est choisie dans le groupe consistant en une tumeur rénale et des tumeurs des organes reproducteurs, notamment un carcinome des ovaires, un cancer du col de l'utérus et un carcinome des testicules.

4. Méthode suivant l'une quelconque des revendications 1 à 3, **caractérisée en ce que** l'échantillon de fluide corporel est un échantillon de sang, un échantillon de plasma ou un échantillon d'une fraction de plasma.

5. Méthode suivant l'une quelconque des revendications 1 à 4, **caractérisée en ce que** ladite valeur de référence est comprise entre 10 et 18 mg, de préférence 13 à 15 mg, de ApoA-IV/dl de sérum.

6. Méthode suivant l'une quelconque des revendications 1 à 5, **caractérisée en ce que** la tumeur est diagnostiquée lorsque le taux de ApoA-IV est inférieur à 10, de préférence inférieur à 8 mg de ApoA-IV/dl de sérum.

7. Méthode suivant l'une quelconque des revendications
1 à 6, **caractérisée en ce que** la teneur en ApoA-IV de l'échantillon est réduite, si elle est inférieure d'au moins 10 %, de préférence inférieure d'au moins 30 %, en particulier inférieure d'au moins 50 % par rapport à la teneur en ApoA-IV d'un échantillon provenant d'un individu dépourvu de tumeur.

8. Méthode suivant l'une quelconque des revendications 1 à 7, **caractérisée en ce que** la quantité de ApoA-IV de l'échantillon est déterminée au moyen d'une méthode immunochimique utilisant des anticorps dirigés contre l'ApoA-IV.

9. Méthode suivant la revendication 8, **caractérisée en ce que** lesdits anticorps sont des anticorps monoclonaux.

10. Méthode suivant la revendication 8 ou 9, **caractérisée en ce que** ledit anticorps comprend un marqueur de détection, de préférence un marqueur chromogène, fluorogène ou radioactif.

11. Méthode suivant l'une quelconque des revendications 8 à 10, **caractérisée en ce que** ladite méthode immunochimique est choisie dans le groupe consistant en une analyse de transfert d'empreintes Western, une analyse par immunosorbant lié à une enzyme 20(ELISA) et une analyse radio-immunologique (RIA).

12. Utilisation d'une méthode suivant l'une quelconque des revendications 1 à 11, pour le contrôle de l'efficacité d'un traitement d'une tumeur.

13. Utilisation suivant la revendication 12, dans laquelle ladite tumeur est choisie dans le groupe consistant en une tumeur rénale et des tumeurs des organes reproducteurs, notamment un carcinome des ovaires, un cancer du col de l'utérus et un carcinome des testicules.

14. Kit pour le diagnostic d'une tumeur chez un patient ou pour le contrôle de l'efficacité d'un traitement d'une tumeur, comprenant un ou plusieurs des constituants suivantes :
- un témoin positif qui est un échantillon provenant d'un individu souffrant d'une tumeur,
- un moyen pour mesurer la quantité d'ApoA-IV dans un échantillon de fluide corporel ou de tissu, ledit moyen pour mesurer la quantité d'AoA-IV étant choisi entre des anticorps anti-ApoA-IV, notamment des anticorps poly-clonaux, des anticorps secondaires, notamment des anticorps secondaires marqués enzymatiquement ou chimiquement, des 5acides nucléiques spécifiques de l'ARN de ApoA-IV, des tests enzymatiques spécifiques de ApoA-IV, des analyses ELISA spécifiques d'ApoA-IV ou leurs associations, et
- un échantillon provenant d'un individu ne souffrant
pas de ladite tumeur, comme moyen d'obtention d'une valeur de référence.

15. Kit suivant la revendication 14, **caractérisé en ce que** le témoin positif et l'échantillon provenant d'un individu ne souffrant pas de ladite tumeur sont stabilisés, de préférence stabilisés par lyophilisation.
